(19) Europäisches Patentamt
European Patent Office
Office européen des brevets

(11) Publication number: **0 124 925**
**B1**

(12) # EUROPEAN PATENT SPECIFICATION

(45) Date of publication of patent specification: **26.11.86**

(51) Int. Cl.⁴: **C 07 C 153/017, A 61 K 31/265**

(21) Application number: **84200478.0**

(22) Date of filing: **05.04.84**

(54) **Derivatives of d-2-(6-methoxy-2-naphthyl)-propionic acid having therapeutical activity, process for their preparation and pharmaceutical compositions containing them.**

(30) Priority: **11.04.83 IT 2052383**

(43) Date of publication of application:
**14.11.84 Bulletin 84/46**

(45) Publication of the grant of the patent:
**26.11.86 Bulletin 86/48**

(84) Designated Contracting States:
**AT BE CH DE FR LI**

(56) References cited:
**FR-A- 941 289**
**GB-A-2 054 586**
**US-A-4 328 242**

**Chemical Abstracts vol. 94, no. 23, 1981, Columbus, Ohio, USA page 701, column 2, abstract no. 192703s**

(73) Proprietor: **FARMA RESA SrL**
**Corso Venezia 61**
**I-20100 Milano (IT)**

(72) Inventor: **Franchina, Antonino**
**Via Flaminia 388**
**I-00100 Roma (IT)**

(74) Representative: **Dragotti, Gianfranco**
**SAIC BREVETTI s.a.s. Viale Bianca Maria, 15**
**I-20122 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 124 925 B1

**Description**

The present invention relates to derivatives of d-2-(6-methoxy-2-naphthyl)propionic acid having therapeutical activity, the process for their preparation and the pharmaceutical compositions containing them.

The invention particularly relates to derivatives of d-2-(6-methoxy-naphthyl)propionic acid having general formula:

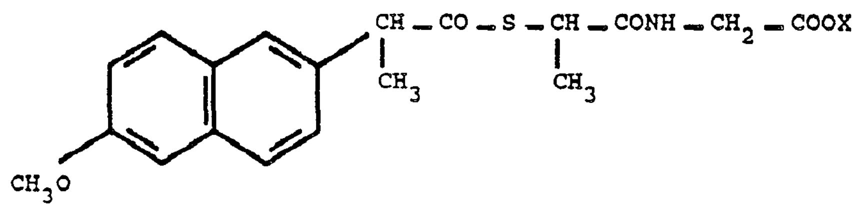

wherein X is selected from the group comprising H, and a radical selected among the radicals of organic or inorganic, non toxic and pharmaceutically acceptable bases, the radicals of basic aminoacids and the radicals of basic antibiotics. As it is known, the d-2-(6-methoxy-2-naphthyl propionic acid (Naproxen) is endowed with anti-inflammatory, analgesic and antipyretic, pharmacologically well demonstrated properties and, as such, this acid found clinical use in the therapy of rheumatoid arthritis and of other degenerative forms having a phlogistic feature.

The alpha-mercaptopropionylglycine is in turn a compound known as well, possessing, besides some anti-inflammatory activity, a relevant mucolytic activity.

The main purpose of the present invention is that of providing novel derivatives of d-2-(6-methoxy-2-naphthyl)-propionic acid, which, in comparison with the starting compounds, are endowed with an improved anti-inflammatory, analgesic, antipyretic and mucolytic activity.

Another purpose of the present invention is that of providing a process for the preparation of these derivatives.

A further purpose of the present invention is that of providing pharmaceutical compositions particularly useful for the therapeutical indications in which the aforesaid properties are particularly advantageous.

The invention relates particularly to derivatives of d-2-(6-methoxy-2-naphthyl-propionic acid permitting the above mentioned purposes to be attained, having the general formula:

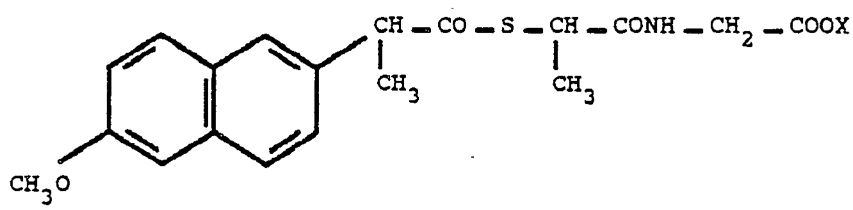

wherein X is selected in the group comprising H, and a radical selected among the radicals of organic or inorganic, non toxic and pharmaceutically acceptable bases, the radicals of basic aminoacids and the radicals of basic antibiotics.

The process according to the invention does essentially comprise the following steps:

a) preparation of a reactive derivative, particularly chloride or mixed anhydride with ethyl chloroformiate, of the d-2-(6-methoxy-2-naphthyl)-propionic acid:

b) reaction of said reactive derivative of the d-2-(6-methoxy-2-naphthyl-propionic acid with an alkaline salt of alpha-mercaptopropionylglycine;

c) isolation of the pure DL form of the thio-derivative obtained in the preceding step; and

d) optional salification of the pure DL derivative.

Among the organic bases useful for the purposes of the present invention there can be included: primary and secondary amines, such as for instance diethylamine, and the primary, secondary and tertiary aminoalcohols, such as for example diethanolamine. Among the inorganic bases suitable for the invention there are contemplated sodium hydroxide, potassium hydroxide and silver hydroxide.

Examples of basic aminoacids useful for the invention are arginine, lysine and histidine, whereas examples of basic antibiotics useful for the purposes of the invention are represented by erythromycin, propionylerythromycin, mechlocycline, clindamycin and josamycine.

Among the derivatives of the invention there are for example included:

Sodium salt of DL-2-(6-methoxy-2-naphthyl)-propionyl-alpha-thio-propionylglycine;

Potassium salt of DL-2-(6-methoxy-2-naphthyl)-propionyl-alpha-thio-propionylglycine;

Silver salt of DL-2-(6-methoxy-2-naphthyl)-propionyl-alpha-thio-propionylglycine;
Lysine salt of DL-2-(6-methoxy-2-naphthyl)-propionyl-alpha-thio-propionylglycine;
Arginine salt of DL-2-(6-methoxy-2-naphthyl)-propionyl-alpha-thio-propionylglycine;
Histidine salt of DL-2-(6-methoxy-2-naphthyl)-propionyl-alpha-thio-propionylglycine;
Erythromycin salt of DL-2-(6-methoxy-2-naphthyl)-propionyl-alpha-thio-propionylglycine;
Propionylerythromycin salt of DL-2-(6-methoxy-2-naphthyl)-propionyl-alpha-thiopropionylglycine;
Mechlocycline salt of DL-2-(6-methoxy-2-naphthyl)-propionyl-alpha-thio-propionylglycine;
Clindamycin salt of DL-2-(6-methoxy-2-naphthyl)-propionyl-alpha-thio-propionylglycine;
Josamycine salt of DL-2-(6-methoxy-2-naphthyl)-propionyl-alpha-thio-propionylglycine.

According to a preferred embodiment of the process of the present invention the chlorination of the d-2-(6-methoxy-2-naphthyl)-propionic acid is carried out with thionyl chloride in the presence of a solvent (preferably chloroform). From the chlorination step crystals of chloride are obtained which are used as such for the subsequent step. According to the invention an alkaline solution of the sodium salt of alpha-mercaptopropionylglycine is prepared, which is slowly added (preferably over about 4 hours) with a solution of the chloride of d-2-(6-methoxy-2-naphthyl)-propionic acid as prepared in the preceding step.

This solution is preferably obtained by dissolving the chloride crystal into dioxane.

The reaction is carried out at temperature of between 0 and 5°C and through acidification (preferably with HCl) a precipitate of d-2-(6-methoxy-2-naphthylpropionyl-alpha-thio-propionylglycine is obtained.

The isolation of the pure DL form of the thio-derivative obtained as above described is carried out, according to the invention, by dissolving the thio-ester in a suitable solvent, for example ethylacetate and by subsequent slow crystallization.

The process of the invention lastly comprises the possible salification of the DL-2-(6-methoxy-2-naphthyl)-propionyl-alpha-thio-propionylglycine as obtained from the preceding crystallization step.

Such a salification can be obtained through the reaction of the pure DL-thio-derivative with an organic or inorganic base, a basic aminoacid or with a basic antibiotic, such as of the previously said type, in the presence of a solvent.

Suitable solvents are for instance water, alcohols (e.g. ethanol) and ketones (e.g. acetone).

The salification reaction is normally carried out by stirring the reactants and then through cold precipitation of the desired salt.

The following example has the purpose of better illustrating the invention, without however limiting the scope thereof.

Example

a) *Chlorination of the d-2-(6-methoxy-2-naphthyl)-propionic acid*

The chlorination is carried out by putting 50 g of d-2-(6-methoxy-2-naphthyl)-propionic acid in 200 ml of chloroform with 100 ml of thionyl chloride.

The mixture is heated to reflux until the development of hydrogen chloride and of sulfurous anhydride ceases.

When the gas development ceases, the mixture is cooled and then concentrated under vacuum to dryness.

The product is taken with the minimum amount of chloroform and then the chloride of the d-2-(6-methoxy-2-naphthyl)-propionic acid is crystallized, filtered and dried.

It is used as such for the next step.

b) *Preparation of d-2-(6-methoxy-2-naphthyl)-propionyl-alpha-thio-propionylglycinic acid*

A 2000 ml flask is charged with 230 g of alpha-mercaptopropionylglycine dissolved in one liter of water.

This solution is added with 56 g of NaOH in form of flakes and then the solution is cooled to a temperature of between 0°C and 15°C.

The solution obtained is made alkaline by adding another amount of NaOH as above.

To this solution is then added a dioxane solution in which the chloride of d-2-(6-methoxy-2-naphthyl)-propionic acid is dissolved.

Such an addition takes place slowly over about 4 hours at temperatures of between 0 and 5°C.

When the addition is completed, the mixture is maintained under stirring for more than 10 hours.

Thereafter the whole mixture is treated with 37% HCl until completely acidic. A white product precipitates which is filtered and washed with $H_2O$ and then dried.

A yield of about 80% of the theoretical value is obtained of a white product having melting point 135—138°C, with a chromatographic purity, as evaluated using chloroform/acetic acid/water (85/15/1) as eluent and Merk 5735 plates, corresponding to an Rf value of 0.61.

c) *Separation with ethyl acetate through crystallization of an optical DD form from the DL form obtained in the preparation of the d-2-(6-methoxy-2-naphthyl)-propionyl-alpha-thio-propionylglycinic acid.*

The product obtained in the step (b), is charged as such (400 g) in a flask provided with a stirrer, and is treated with ethyl acetate by heating the solution to reflux, using 850 ml of solvent.

The mixture is then slowly crystallized for 24 hours and there are obtained 120 g of a white crystalline

product, corresponding to DL-2-(6-methoxy-2-naphthyl-propionyl-alpha-thio-propionylglycine, having melting point 175—177°C, and a rotatory index $[alpha]_D^{20}=0°$

d1) *Preparation of L(+)lysine salt of DL-2-(6-methoxy-2-naphthyl)-propionyl-alpha-thio-propionylglycine acid.*

It is obtained in ethanol from 11.3 g of DL-2(6-methoxy-2-naphthyl)-propionyl-alpha-thio-propionyl glycinic acid and 4.5 g of crystals of lysine base.

The ethanolic solution is heated to reflux and then cooled and there are obtained 12.5 g of L(+)-lysine salt of the DL-2-(6-methoxy-2-naphthyl)-alpha-thio-propionyl glycinic acid, having melting point 175—180°C and which is perfectly soluble in water.

d2) *Preparation of the erythromycin salt of DL-2-(6-methoxy-2-naphthyl)-propionyl-alpha-thio-propionylglycinic acid.*

The reaction takes place in aqueous acetonic medium by charging in the flask under stirring 1 mole of DL-2-(6-methoxy-2-naphthyl-propionyl-alpha-thio-propionyl glycinic acid and then adding 1 mole of pure erythromycin base.

The whole is maintained under stirring and by cooling to a temperature of between 0 and 10°C the salt precipitates with a yield of 75% of the theoretical value.

The product is filtered, well washed with a 50% acetone/water solution and then dried.

By the same process as in the preceding example the salt with base DL-lysine or with arginine can be obtained (step d1); the arginine crystalline product obtained in that step is soluble in water with pH=7.3 and has a definite melting point of 178—179°C.

By a like process (step d2), the salt with propionylerythromycin can be obtained in form of crystals with a melting point 111—113°C.

The compounds of the present invention have been subjected to preliminary pharmacological tests, and those relating to the propionylerythromycin salt of DL-2-(6-methoxy-2-naphthyl)-propionyl-alpha-thio-propionylglycine (hereinafter shortly indicated with the abbreviation NXTP) are hereinafter reported.

A) *Acute toxicity*

The acute toxicity both in the mouse and the rat was evaluated per os and by intraperitoneal route, the following results being obtained:

1) in the mouse:

| | |
|---|---|
| per os | $LD_{50}$>1,500 mg/kg |
| i.p. | $LD_{50}$=405 mg/kg |

2) in the rat:

| | |
|---|---|
| per os | $LD_{50}$1,500 mg/kg |
| i.p. | $LD_{50}$>1,500 mg/kg |

B) *Anti-inflammatory activity*

This activity has been evaluated for the NXTP compound by the carrageenin induced oedema test, carried out in the manner known in the literature, in Sprague-Dawley male rats, having a body weight of 180—200 g, the NXTP being administered to groups of 3 animals each at the dosages of 50, 100, 200 and 400 mg/kg, the results being evaluated in comparison with a control group.

The volume increase of the paw in the group of control rats was on the average 60.30% and it was observed that the tested compound causes the oedema to be inhibited proportionally to the administered dose. Since in no case an inhibition rate of 50% was attained, the value of $ED_{33}$ was calculated, it being 759 mg/kg (0,651 mM/kg).

C) *Analgesic activity*

This test was carried out in Swiss male albino mice, having a body weight of 30—40 g through the "stretching or writhing" inhibition test, as induced by acetic acid, the test being carried out in a manner per se known from the literature.

It was found that the NXTP caused an inhibition of the number of contorsions induced by acetic acid in a rate proportional to the administered doses (50,100 and 200 mg/kg as injected by subcutaneous route). The $ED_{50}$ of NXTP was 400 mg/kg.

D) *Antipyretic activity*

This test has been carried out in the manner known from the literature in Sprague-Dawley male rats,

having a body weight of 190—220 g, which were selected according to the rectal temperature which was between 37.0 and 37.7°C.

The hyperpyrexy was induced by subcutaneous injection of 2.0 ml of a 7.5% suspension of yeast as prepared at the moment.

The tested dosages were 100, 200 and 400 mg/kg and the found $ED_{33}$ value was 802 mg/kg.

E) *Mucolytic activity*

This test was carried out, in the manner known from the literature, in 13 ml samples of expectorate collected from hospitalized patients, and stored in a freezer at −20°C until the testing time.

The viscosity of the expectorate added with 1 ml of physiological solution was at the beginning 15.5 and after one hour incubation at 37°C showed a 13% reduction (value of the viscosity at t=1h equal to 13.5). At the tested concentration of the compound undergoing the experiment (0.05M) a viscosity reduction of 16% (29% to 13%) was found.

As above stated, the invention also relates to the pharmaceutical compositions containing as the active principle the above described derivatives, in admixture with solid, liquid or semiliquid, physiologically compatible excipients, which can be administered by parenteral, oral, topical or rectal route.

The compositions which can be normally administered by oral route include tablets, pills, gastroeresistant tablets, powders for the prompt preparation of syrups and lastly granulates.

As the excipients those normally used in the related art are contemplated, such as for instance talc, starch, lactose.

These compositions for oral use can be obviously also admixed with lubricating agents, such as magnesium stearate. Among the compositions of liquid type for oral administration, syrups, emulsions, suspensions and elixirs, duly sweetened and flavoured are meant.

As the liquid vehicles water, paraffin and hydro-alcoholic solutions can be used. Furthermore for the oral administration also capsules with a solid or semiliquid content are foreseen.

For the administration by parenteral route, as the vehicle distilled water and propylene glycol, polyethylene glycol, vegetable oils and their organic esters, such as for instance ethyl oleate, are meant.

All these injectable formulations can be sterilized by means of filtering membranes or sterilizing agents, both of chemical and of physical nature.

For the administration by rectal route the normal suppositories or absorbable capsules are meant, having as the vehicle cacao butter, stearates and waxes.

As regards the therapeutical administration of the compositions of the present invention, dosages equivalent to those normally used for the starting acid, namely of d-2-(6-methoxy-2-naphthyl)-propionic acid, are contemplated, with the advantages deriving for instance from the addition of the anti-inflammatory and mucolytic activity specific of the alpha-mercapto-propionylglycine, as well as of the possible specific activity of the antibiotic when the latter is used for the salification.

**Claims**

1. Therapeutically active derivatives of d-2-(6-methoxy-2-naphthyl)-propionic acid having general formula:

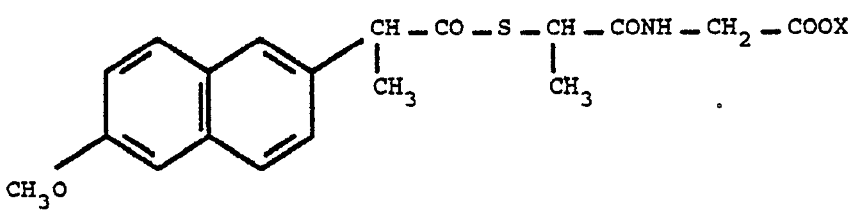

wherein X is selected from the group comprising H, and a radical selected among the radicals of organic or inorganic, non toxic and pharmaceutically acceptable bases, the radicals of basic aminoacids and the radicals of basic antibiotics.

2. Therapeutically active derivatives according to claim 1, characterized in that said organic bases are selected among primary and secondary amines and among primary, secondary and tertiary aminoalcohols.

3. Therapeutically active derivatives according to claim 2, characterized in that said organic bases are selected among diethanolamine and diethylamine.

4. Therapeutically active derivatives according to claim 1, characterized in that said inorganic bases are selected among sodium, potassium and silver hydroxides.

5. Therapeutically active derivatives according to claim 1, characterized in that said basic aminoacids are selected among arginine, lysine and histidine.

6. Therapeutically active derivatives according to claim 1, characterized in that said basic antibiotics are selected among erythromycin, propionylerythromycin, mechlocycline, clindamycine and josamycine.

7. Sodium salt of DL-2-(6-methoxy-2-naphthyl)-propionyl-alpha-thio-propionylglycinic acid according to claim 1.

8. Potassium salt of DL-2-(6-methoxy-2-naphthyl)-propionyl-alpha-thio-propionylglycinic acid according to claim 1.

9. Silver salt of DL-2-(6-methoxy-2-naphthyl)-propionyl-alpha-thio-propionylglicinic acid according to claim 1.

10. Lysine salt of DL-2-(6-methoxy-1-naphthyl-propionyl-alpha-thio-propionylglycinic acid according to claim 1.

11. Arginine salt of DL-2-(6-methoxy-2-naphthyl)-propionyl-alpha-thio-propionylglycinic according to claim 1.

12. Histidine salt of DL-2-(6-methoxy-2-naphthyl)-propionyl-alpha-thio-propionylglycinic acid according to claim 1.

13. Erythromycin salt of DL-2-(6-methoxy-2-naphthyl)-propionyl)-alpha-thio-propionylglycinic acid according to claim 1.

14. Propionylerythromycin salt of DL-2-(6-methoxy-2-naphthyl)-propionyl-alpha-thio-propionylglycinic acid according to claim 1.

15. Mechlocycline salt of DL-2-(6-methoxy-2-naphthyl)-propionyl-alpha-thio-propionylglycinic acid according to claim 1.

16. Clindamycin salt of DL-2-(6-methoxy-2-naphthyl)-propionyl-alpha-thio-propionylglycinic acid according to claim 1.

17. Josamycine salt of DL-2-(6-methoxy-2-naphthyl)-propionyl-alpha-thio-propionylglycinic acid according to claim 1.

18. A process for the preparation of the derivatives according to the preceding claims characterized by comprising the steps of:

a) preparation of a reactive derivative of d-2-(6-methoxy-2-naphthyl)-propionic acid;

b) reaction of said reactive derivative with an alkaline salt of alpha-mercaptopropionylglycine;

c) isolation of the pure DL form of the thus obtained thio-derivative; and

d) optional salification of the DL-thio-derivative.

19. A process according to claim 18, characterized in that said reactive derivative is selected among the chloride and the mixed anhydride, e.g. with ethyl chloroformate, of the d-2-(6-methoxy-2-naphthyl-propionic acid, and in that said alkaline salt is the sodium salt of alpha-mercaptopropionylglycine.

20. A process according to claim 19, characterized in that the chlorination of the d-2-(6-methoxy-2-naphthyl)propionic acid is carried out by adding thionyl chloride, in the presence of a solvent.

21. A process according to claim 20, characterized in that the reaction of the chloride of d-2-(6-methoxy-2-naphthyl-propionic acid with the sodium salt of alpha-mercaptopropionylglycine is carried out in aqueous medium at a temperature of between 0°C and +5°C.

22. Pharmaceutical composition for parenteral, oral, rectal or topical use, containing a therapeutically active amount of a derivative according to any of claims 1 to 5.

**Patentansprüche**

1. Therapeutisch aktive Derivate von d-2(6-Methoxy-2-naphthyl)-propionsäure mit der allgemeinen Formel

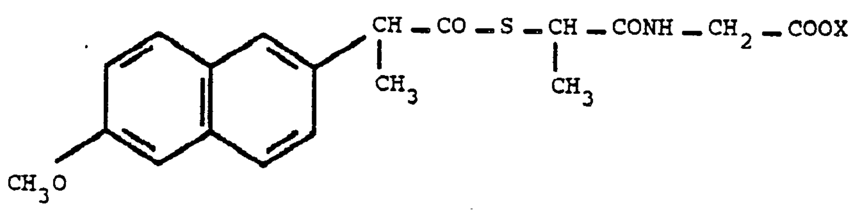

worin X aus der Gruppe ausgewählt ist, die H und ein Radikal umfaßt, welches aus Radikalen von organischen oder anorganischen nichttoxischen und pharmazeutisch annehmbaren Basen, den Radikalen von basischen Aminosäuren und den Radikalen von basischen Antibiotika, ausgewählt ist.

2. Therapeutisch aktive Derivate nach Anspruch 1, dadurch gekennzeichnet, daß die genannten organischen Basen aus primären und sekundären Aminen und aus primären, sekundären und tertiären Aminoalkoholen ausgewählt sind.

3. Therapeutisch aktive Derivate nach Anspruch 2, dadurch gekennzeichnet, daß die genannten organischen Basen aus Diethanolamin und Diethylamin ausgewählt sind.

4. Therapeutisch aktive Derivate nach Anspruch 1, dadurch gekennzeichnet, daß die genannten organischen Basen aus Natrium-, Kalium- und Silberhydroxiden ausgewählt sind.

5. Therapeutisch aktive Derivate nach Anspruch 1, dadurch gekennzeichnet, daß die genannten basischen Aminosäuren aus Arginin, Lysin und Histidin ausgewählt sind.

6. Therapeutisch aktive Derivate nach Anspruch 1, dadurch gekennzeichnet, daß die genannten

basischen Antibiotika aus Erythromycin, Propionylerythromycin, Mechlocyclin, Clindamycin und Josamycin ausgewählt sind.

7. Natriumsalz von DL-2-(6-Methoxy-2-naphthyl)-propionyl-alpha-thio-propionylglycinsäure gemäß Anspruch 1.

8. Kaliumsalz von DL-2-(6-Methoxy-2-naphthyl)-propionyl-alpha-thio-propionylglycinsäure gemäß Anspruch 1.

9. Silbersalz von DL-2-(6-Methoxy-2-naphthyl)-propionyl-alpha-thio-propionylglycinsäure gemäß Anspruch 1.

10. Lysinsalz von DL-2-(6-Methoxy-2-naphthyl)-propionyl-alpha-thio-propionylglycinsäure gemäß Anspruch 1.

11. Argininsalz von DL-2-(6-Methoxy-2-naphthyl)-alpha-thio-propionylglycinsäure nach Anspruch 1.

12. Histidinsalz von DL-2-(6-Methoxy-2-naphthyl)-propionyl-alpha-thio-propionylglycinsäure nach Anspruch 1.

13. Erythromycinsalz von DL-2-(6-Methoxy-2-naphthyl)propionyl-alpha-thio-propionylglycinsäure nach Anspruch 1.

14. Propionylerythromycinsalz von DL-2-(6-Methoxy-2-naphthyl)-propionyl-alpha-thio-propionylglycinsäure nach Anspruch 1.

15. Mechlocyclinsalz von DL-2-(6-Methoxy-2-naphthyl)-propionyl-alpha-thio-propionylglycinsäure nach Anspruch 1.

16. Clindamycinsalz von DL-2-(6-Methoxy-2-naphthyl)-propionyl-alpha-thio-propionylglycinsäure nach Anspruch 1.

17. Josamycinsalz von DL-2-(6-Methoxy-2-naphthyl)-propionyl-alpha-thio-propionylglycinsäure nach Anspruch 1.

18. Verfahren zur Herstellung der Derivate nach den vorstehenden Ansprüchen, gekennzeichnet durch die Stufen:

a) Herstellung eines reaktiven Derivats von d-2-(6-Methoxy-2-naphthyl)-propionsäure,

b) Umsetzung des genannten reaktiven Derivats mit einem Alkalisalz von alpha-Mercaptopropionylglycin,

c) Isolierung der reinen DL-Form des so erhaltenen Thioderivats, und

d) gegebenenfalls Salzbildung des DL-Thioderivats.

19. Verfahren nach Anspruch 18, dadurch gekennzeichnet, daß man das genannte reaktive Derivat aus dem Chlorid und dem gemischten Anhydrid, z.B. mit Ethylchloroformiat der d-2-(6-Methoxy-2-naphthyl)-propionsäure auswählt und daß das genannte Alkalisalz das Natriumsalz von alpha-Mercaptopropionylglycin ist.

20. Verfahren nach Anspruch 19, dadurch gekennzeichnet, daß man die Chlorierung der d-2-(6-Methoxy-2-naphthyl)-propionsäure durch Zugabe von Thionylchlorid in Gegenwart eines Lösungsmittels durchführt.

21. Verfahren nach Anspruch 20, dadurch gekennzeichnet, daß man die Umsetzung des Chlorids der d-2-(6-Methoxy-2-naphthyl)-propionsäure mit dem Natriumsalz von alpha-Mercaptopropionylglycin in einem wäßrigen Medium bei einer Temperatur von zwischen 0°C und +5°C durchführt.

22. Pharmazeutische Zubereitung für die parenterale, orale, rektale oder topische Anwendung, enthaltend eine therapeutisch aktive Menge eines Derivats nach einem der Ansprüche 1 bis 5.

**Revendications**

1. Dérivés thérapeutiquement actifs de l'acide D-2-(6-méthoxy-2-naphthyl)-propionique de formule générale:

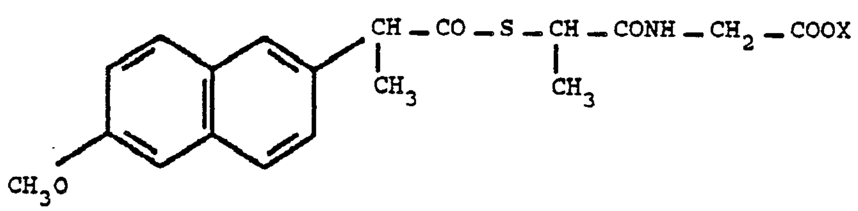

dans laquelle X est choisi dans le groupe comprenant H et un radical selectionné parmi les radicaux des bases organiques ou minérales, non toxiques et pharmaceutiquement acceptables, les radicaux des aminoacides basiques et les radicaux des antibiotiques basiques.

2. Dérivés thérapeutiquement actifs, selon la revendication 1, caractérisés en ce que lesdites bases organiques sont choisies parmi les amines primaires et secondaires et parmi les aminoalcools primaires, secondaires et tertiaires.

3. Dérivés thérapeutiquement actifs selon la revendication 2, caractérisés en ce que lesdites bases organiques sont choisies parmi la diéthanolamine et la diéthylamine.

4. Dérivés thérapeutiquement actifs selon la revendication 1, caractérisés en ce que lesdites bases minérales sont choisies parmi les hydroxydes de sodium, de potassium et d'argent.

5. Dérivés thérapeutiquement actifs selon la revendication 1, caractérisés en ce que lesdits aminoacides basiques sont choisis parmi l'arginine, la lysine et l'histidine.

6. Dérivés thérapeutiquement actifs selon la revendication 1, caractérisés en ce que lesdits antibiotiques basiques sont choisis parmi l'érythromycine, la propionylérythromycine, la méchlocycline, la clindamycine et la josamycine.

7. Sel de sodium de l'acide DL-2-(6-méthoxy-2-naphthyl)-propionyl-alpha-thio-propionylglycinique selon la revendication 1.

8. Sel de potassium de l'acide DL-2-(6-méthoxy-2-naphthyl)-propionyl-alpha-thio-propionylglycinique selon la revendication 1.

9. Sel d'argent de l'acide DL-2-(6-méthoxy-2-naphthyl)-propionyl-alpha-thio-propionylglycinique selon la revendication 1.

10. Sel de lysine de l'acide DL-2-(6-méthoxy-2-naphthyl)-propionyl-alpha-thio-propionylglycinique selon la revendication 1.

11. Sel d'arginine de l'acide DL-2-(6-méthoxy-2-naphthyl)-propionyl-alpha-thio-propionylglycinique selon la revendication 1.

12. Sel d'histidine de l'acide DL-2-(6-méthoxy-2-naphthyl)-propionyl-alpha-thio-propionylglycinique selon la revendication 1.

13. Sel d'érythromycine de l'acide DL-2-(6-méthoxy-2-naphthyl)-propionyl-alpha-thio-propionylglycinique selon la revendication 1.

14. Sel de propionylérythromycine de l'acide DL-2-(6-méthoxy-2-naphthyl)-propionyl-alpha-thio-propionylglycinique selon la revendication 1.

15. Sel de méchlocycline de l'acide DL-2-(6-méthoxy-2-naphthyl)-propionyl-alpha-thio-propionylglycinique selon la revendication 1.

16. Sel de clindamycine de l'acide DL-2-(6-méthoxy-2-naphthyl)-propionyl-alpha-thio-propionylglycinique selon la revendication 1.

17. Sel de josamycine de l'acide DL-2-(6-méthoxy-2-naphthyl)-propionyl-alpha-thio-propionylglycinique selon la revendication 1.

18. Un procédé de préparation des dérivés selon les revendications précédentes, caractérisé en ce qu'il comporte les étapes suivantes:

a) préparation d'un dérivé réactif de l'acide D-2-(6-méthoxy-2-naphthyl)-propionique;

b) réaction dudit dérivé réactif avec un sel alcalin de l'alpha-mercaptopropionylglycine;

c) isolation de la forme DL pure, du dérivé thio ainsi obtenu et

d) salification éventuelle de la forme DL du dérivé thio.

19. Un procédé selon la revendication 18, caractérisé en ce que ledit dérivé réactif est sélectionné parmi le chlorure et l'anhydride mixte, notamment avec le chloroformiate d'éthyle de l'acide DL-2-(6-méthoxy-2-naphthyl)-propionyl-alpha-thio-propionylglycinique et en ce que ledit sel alcalin est le sel de sodium de l'alpha-mercaptopropionylglycine.

20. Un procédé selon la revendication 19, caractérisé en ce que la chloruration de l'acide D-2-(6-méthoxy-2-naphthyl)-propionique est effectuée en ajoutant du chlorure de thionyle en présence d'un solvant.

21. Un procédé selon la revendication 20, caractérisé en ce que la réaction du chlorure de l'acide D-2-(6-méthoxy-2-naphthyl)-propionique avec le sel de sodium de l'alpha-mercaptopropionylglycine est effectuée, en milieu aqueux, à une température comprise entre 0°C et + 5°C.

22. Composition pharmaceutique à utiliser par voie parentérale, orale ou rectale, ou en application locale, contenant une quantité thérapeutiquement active d'un dérivé selon l'une des revendications 1 à 5.